# EUROPÄISCHE PATENTSCHRIFT

(11) **EP 1 131 301 B1**
(45) Veröffentlichungstag und Bekanntmachung des Hinweises auf die Patenterteilung: **24.08.2005**
(21) Anmeldenummer: 99972206.9
(22) Anmeldetag: 05.11.1999
(51) Int. Cl.: C07D 235/18, A61K 31/4184, C07D 209/14, A61K 31/404, A61P 25/00

(54) **2-PHENYLBENZIMIDAZOLE UND 2-PHENYLINDOLE, DEREN HERSTELLUNG UND ANWENDUNG**
2-PHENYLBENZIMIDAZOLES AND 2-PHENYLINDOLES, AND PRODUCTION AND USE THEREOF
2-PHENYLBENZIMIDAZOLES ET 2-PHENYLINDOLES, LEUR PRODUCTION ET LEUR UTILISATION

(30) Priorität: 17.11.1998 DE 19852816
(43) Veröffentlichungstag der Anmeldung: 12.09.2001
(73) Patentinhaber: Abbott GmbH & Co. KG, 65205 Wiesbaden (DE)
(72) Erfinder: LUBISCH, Wilfried, D-69115 Heidelberg (DE); KOCK, Michael, D-67105 Schifferstadt (DE); HÖGER, Thomas, D-68535 Edingen-Neckarhausen (DE)
(74) Vertreter: Grünecker, Kinkeldey, Stockmair & Schwanhäusser Anwaltssozietät
(86) Internationale Anmeldenummer: PCT/EP1999/008466
(87) Internationale Veröffentlichungsnummer: WO 2000/029384

(56) Entgegenhaltungen:
- WO-A-97/04771
- WO-A-98/06703
- DE-A- 3 522 230
- GILCHRIST T L: "CYCLISATION OF ORTHO-SUBSTITUTED N-ARYLBENZIMIDOYL NITRENES. PART 2.1 PREFERENTIAL CYCLISATIONS AT AN ORTHO-POSITION BEARING A METHOXYCARBONYL GROUP" JOURNAL OF THE CHEMICAL SOCIETY, PERKIN TRANSACTIONS 1,GB,CHEMICAL SOCIETY. LETCHWORTH,1. Januar 1979 (1979-01-01), Seiten 2303-2307, XP000605168 ISSN: 0300-922X in der Anmeldung erwähnt

## Beschreibung

Die vorliegende Erfindung betrifft neuartige 2-Phenylbenzimidazole und 2-Phenylindole, ihre Herstellung und die Verwendung als Inhibitoren des Enzyms Poly(ADP-ribose)polymerase oder PARP (EC 2.4.2.30) zur Herstellung von Arzneimitteln.

Poly(ADP-ribose)polymerase (PARP) bzw. wie es auch genannt wird Poly(ADP-ribose)synthase (PARS) stellt ein regulatorisches Enzym dar, das in Zellkernen gefunden wird (K. Ikai et al., *J.Histo-chem. Cytochem*. **1983,** *31*, 1261-1264). Man nimmt an, daß PARP eine Rolle bei der Reparatur von DNA-Brüchen spielt (M.S. Satoh et al., *Nature* **1992**, *356*, 356-358). Schädigungen oder Brüche der DNA-Stränge aktivieren das Enzym PARP, das, wenn es aktiviert ist, die Übertragung von ADP-Ribose aus NAD katalysiert (S. Shaw, *Adv.Radiat.Biol.,* **1984**, *11*, 1-69). Dabei wird Nikotinamid aus NAD freigesetzt. Nikotinamid wird unter Verbrauch des Energieträgers ATP von anderen Enzymen wieder in NAD umgewandelt. Eine Überaktivierung von PARP hätte dementsprechend einen unphysiologisch hohen Verbrauch von ATP zur Folge und dies führt im Extremfall zu Zellschädigungen und Zelltod.

Es ist bekannt, daß Radikale wie Superoxid-Anion, NO und Wasserstoffperoxid in Zellen zu DNA-Schädigungen führen können und damit PARP aktivieren. Die Bildung von großen Mengen an Radikalen wird bei eine Reihe von pathophysiologischen Zuständen beobachtet und man geht davon aus, daß diese Anhäufung von Radikalen zu den beobachteten Zell- bzw Organschäden führen oder beitragen. Dazu zählt von zum Beispiel ischämische Zustände von Organen wie im Schlaganfall, Herzinfarkt (C. Thiemermann et al., *Proc.Natl.A-cad.Sci.USA,* **1997,** *94,* 679-683) oder Ischämie der Nieren, aber auch Reperfusionsschäden wie sie zum Beispiel nach der Lyse von Herzinfarkt auftreten (s. oben : C. Thiemermann et al.). Die Hemmung von dem Enzym PARP könnte demzufolge ein Mittel sein, um diese Schäden zum mindestens zum Teil zu verhindern oder abzumildern. PARP-Inhibitoren könnten somit ein neues Therapieprinzip zur Behandlung von eine Reihe von Krankheiten darstellen.

Das Enzym PARP beeinflußt die Reparatur von DNA-Schäden und könnte somit auch in der Therapie von Krebs-Erkrankungen eine Rolle spielen, da in Kombination mit cytostatisch wirksamen Stoffen ein höheres Wirkpotential gegenüber Tumorgewebe beobachtet wurde (G. Chen et al. *Cancer Chemo. Pharmacol*. **1988,** *22*, 303).

Nicht limitierende Beispiele für Tumoren sind Leukämie, Glioblassome, Lymphome, Melanome, Mama- und Zervikalkarzinome.

Zudem wurde gefunden, daß PARP-Inhibitoren immunosuppressive Wirkung zeigen können (D. Weltin et al. *Int.J.Immunopharmacol*. **1995,** *17*, 265-271).

Es wurde ebenfalls entdeckt, daß PARP bei immunologischen Erkrankungen bzw. Krankheiten, in denen das Immunsystem eine wichtige Rolle spielt, wie zum Beispiel rheumatoide Arthritis und septischer Schock, involviert ist, und daß PARP-Inhibitoren einen günstigen Effekt auf den Krankheitsverlauf zeigen können (H. Kröger et al. *Infammation* **1996,** *20,* 203-215; W.Ehrlich et al. *Rheumatol. Int*. **1995,** *15*, 171-172; C.Szabo et al., *Proc.Natl.Acad.Sci.USA* **1998,** *95*, 3867-3872; S. Cuzzocrea et al. *Eur.J.Pharmacol*. **1998,** *342*, 67-76).

Unter PARP im Sinne dieser Erfindung werden auch Isoenzyme des oben beschriebenen PARP-Enzyms verstanden.

Weiterhin zeigte der PARP-Inhibitor 3-Aminobenzamid protektive Effekte in einem Model für den Kreislaufschock (S. Cuzzocrea et al., *Br.J.Pharmacol*. **1997,** *121*, 1065-1074).

Ebenfalls gibt es experimentelle Hinweise, das Inhibitoren des Enzymes PARP als Mittel zur Behandlung von Diabetes mellitus nützlich sein könnten (V. Burkart et al. *Nature Med.* 1999, 5, 314-319).

2-Phenylbenzimidazole sind vielfach beschrieben worden. So sind in DE 38 30 060 alkylierte Derivate als Inhibitoren der Erythrozytenaggregation offengelegt. In DE 35 22 230 ist ein Ester-Derivat vom 2-Phenylbenzimidazol als Inhibitor der Plättchenaggragation aufgeführt. Halogen-substituierte 2-Phenylbenzimizazole, die am Phenyl-Ring substituierte Amin-Reste tragen, sind in WO 98/06703 als MCP-1-Antagonisten beschrieben worden.

Ebenfalls sind 2-Phenyl-benzimidazole bekannt, bei denen die Benzimidazol-Gruppe durch eine Amid-Gruppe substituiert ist. 5-Amido-Derivate des 2-Phenylbenzimidazols, die am Phenyl-Ring Alkyloxy-Reste tragen, sind in WO 94/12461 als Inhibitoren der cAMP-Phosphodiesterase beschrieben worden. Für analoge Derivate wurde in DE 35 46 575 (z.B. Beispiel 15) gefunden, daß diese Verbindungen positiv inotrope Effekte auslösen. Ebenfalls 4-Amido-Derivate, die in 3-Stellung ein Pyridyl-Rest tragen, sind in WO 97/48697 als Inhibitoren der cAMP-Phosphodiesterase aufgeführt.

Die Synthese von 2-Phenyl-benzimidazyl-4-amiden ist in J.Chem.Soc. Perkin Trans 1, 1979, 2303-2307 beschrieben worden. Analoge Verbindungen, die am Amid-Rest noch eine substituierte Alkyl-Kette tragen, und die cytotoxische Wirkung haben sollen, sind in J.Med.Chem. 1990, 33, 814-819 aufgeführt. In WO 97/04771 sind dagegen Benzimidazol-4-amide aufgeführt, die das PARS hemmen. Insbesondere sind Derivate dort als wirksam beschrieben , die einen Phenyl-Ring in 2-Stellung tragen, wobei der Phenyl-Ring noch mit einfachen Substituenten wie Nitro, Methoxy und CF₃, substituiert sein kann. Obwohl diese Substanzen zum Teil gute Hemmung des Enzyms PARP zeigen, haben die dort beschrieben Derivate als Nachteil, daß sie nur wenig oder keine Löslichkeit in wäßrigen Lösungen zeigen und somit nicht als wäßrige Lösung appliziert werden können.

In einer Reihe von Therapien wie Schlaganfall werden die Wirkstoffe intravenös als Infusionslösung appliziert. Dazu ist es notwendig Substanzen, hier PARP-Inhibitoren, zur Verfügung zu haben, die ausreichende Wasserlöslichkeit bei physiologischen pH-Werten oder angenäherten pH-Werten (z.B: pH-Werten von 5-8) aufweisen, so daß eine Infusionslösung hergestellt werden kann. Viele der beschriebenen PARP-Inhibitoren, insbesondere die besser wirksamen PARP-Inhibitoren, haben jedoch den Nachteil, daß sie nur geringe oder keine Wasserlöslichkeit bei diesen pH-Werten zeigen und somit nicht für eine intravenöse Applikation in Frage kommen. Derartige Wirkstoffe können nur mit Hilfsstoffen, die die Wasserlöslichkeit vermitteln sollen, appliziert werden (vgl WO 97/04771). Diese Hilfsstoffe, zum Beispiel Polyethylenglykol und Dimethysulfoxid, verursachen häufig Nebeneffekte oder sind sogar unverträglich. Gut wirksame PARP-Inhibitoren mit ausreichender Wasserlöslichkeit sind bisher nicht beschrieben worden.

Es wurde überraschender weise gefunden, daß 2-Phenyl-benzimidazole, die am Phenyl-Ring noch einen Amin-Rest tragen, gut wirksame Inhibitoren darstellen, die aber durch den Einbau des aliphatischen Amin-Restes eine Salzbildung mit Säuren ermöglichen und dadurch eine deutlich verbesserte Wasserlöslichkeit zeigen.

In der vorliegenden Erfindung werden neue 2-Phenylbenzimidazol- und 2-Phenylindol-Derivate der allgemeinen Formel I beschrieben, die gegenüber den bereits beschriebenen Verbindungen Vorteile zeigen und potente PARP-Inhibitoren darstellen und zugleich auch ausreichende Wasserlöslichkeit zeigen, die eine Applikation als Infusionlösung ermöglicht.

Gegenstand der vorliegenden Erfindung sind substituierte 2-Phenylbenzimidazole und 2-Phenylindole der allgemeinen Formel I: 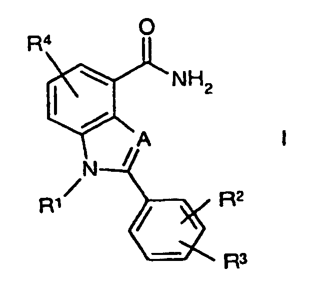 worin
- A: N oder CH bedeutet
- R¹: Wasserstoff, verzweigtes und unverzweigtes C₁-C₆-Alkyl, wobei ein C-Atom des Alkyl-Restes noch OR¹¹ tragen kann, wobei
- R¹¹: Wasserstoff oder C₁-C₄-Alkyl bedeutet, und
- R²: Wasserstoff, Chlor, Fluor, Brom, Jod, verzweigtes und unverzweigtes C₁-C₆-Alkyl, Nitro, CF₃, CN, NR²¹R²², NH-CO-R²³, OR²¹, wobei
- R²¹ und R²²: unabhängig voneinander Wasserstoff oder C₁-C₄-Alkyl bedeuten und
- R²³: Wasserstoff, C₁-C₄-Alkyl oder Phenyl bedeuten, und
- R³: -NR³¹R³², (CH₂)_{q}-NR³³R³⁴ bedeutet, wobei q 1, 2 oder 3 sein kann,
- R³¹: bedeutet Wasserstoff, C₁-C₆-Alkyl, (CH₂)ᵣN³³R³⁴
- R³²: bedeutet (CH₂)ᵣNR³³R³⁴,
worin bei R³¹ und R³² unabhängig voneinander r 2, 3, 4, 5 oder 6 bedeutet und R³³ und R³⁴ unabhängig voneinander Wasserstoff, C₁-C₆-Alkyl, zusammen mit dem Stickstoff gleich einem Ring 3 bis 8 Atomen, der ein zusätzliches Heteroatom ausgewählt aus O, N-C₁-C₄-Alkyl, N-C₀-C₂-Phenyl oder NH tragen kann,
Phenyl-C₁-C₄-Alkyl, wobei der Phenylring mit bis zu 3 gleichen oder verschiedenen Substituenten ausgewählt aus der Gruppe C₁-C₆-Alkyl, Halogen, Nitro, SO₂NR³⁵R³⁶ (mit R³⁵, R³⁶ unabhängig voneinander gleich Wasserstoff, C₁-C₄-Alkyl oder zusammen mit dem Stickstoff gleich einem Ring 3 bis 8 Atomen, der ein zusätzliches Heteroatom ausgewählt aus O, S, SO₂, N-C₁-C₄-Alkyl, N-Co-C₂-Phenyl oder NH tragen kann), C₁-C₄-Alkoxy, S(O)₀₋₂-R³⁷ (mit R³⁷ gleich Wasserstoff, C₁-C₄-Alkyl), CF₃, (CH₂)₀₋₄-COR³⁷, (CH₂)₀₋₄NR³⁵R³⁶, (CH₂)₀₋₄ CONR³⁵R³⁶, (CH₂)₀₋₄OR³⁷-CH₂COOR³⁷,
- R⁴: Wasserstoff, verzweigtes und unverzweigtes C₁-C₆-Alkyl, Chlor, Brom, Fluor, Nitro, Cyano, NR⁴¹R⁴², NH-CO-R⁴³, OR⁴¹, wobei
- R⁴¹ und R⁴²: unabhängig voneinander Wasserstoff oder C₁-C₄-Alkyl bedeuten und
- R⁴³: C₁-C₄-Alkyl oder Phenyl bedeuten, und
sowie ihre tautomeren Formen, möglichen enantiomeren und diastereomeren Formen, deren Phosphate, Carbamate von Aminosäuren, Ester, sowie mögliche physiologisch verträgliche Salze.

Bevorzugte Positionen für den Rest R² in der allgemeinen Formel I sind die 3-Position und die 4-Position zum Benzimidazolring. Für den Rest R³ ist ebenfalls die 3-Position oder 4-Position zum Benzimidazolring bevorzugt.

Die bevorzugte Bedeutung von A ist Stickstoff.

Die bevorzugte Bedeutung von R¹ ist Wasserstoff.

Die bevorzugte Bedeutung von R² ist Wasserstoff, verzweigtes oder unverzweigtes C₁-C₆-Alkyl, Nitro, CN, NH₂, O-C₁-C₄-Alkyl. Besonders bevorzugt ist R² gleich Wasserstoff.

Die bevorzugte Bedeutung für R³ ist (CH₂)_{1,2}NR³⁵R³⁶ und N(R³⁷)-(CH₂)₂₋₃NR³⁵R³⁶, worin R³⁷ Wasserstoff und C₁-C₄-Alkyl sein kann, R³⁵ und R³⁶ unabhängig voneinander Wasserstoff und C₁-C₄-Alkyl und zusammen als NR³⁵R³⁶ auch Piperidin, Pyrrolidin, Azepin und Piperazin sein können, wobei das Piperazin am zweiten N-Atom noch mit Wasserstoff oder C₁-C₄-Alkyl substituiert sein kann.

Die bevorzugte Bedeutung von R⁴ ist Wasserstoff.

Ganz besonders bevorzugt sind die jeweiligen Kombinationen der obigen bevorzugten Bedeutungen.

Die Verbindungen der Formel I können als Racemate, als enantiomerenreine Verbindungen oder als Diastereomere eingesetzt werden. Werden enantiomerereine Verbindungen gewünscht, kann man diese beispielsweise dadurch erhalten, daß man mit einer geeigneten optisch aktiven Base oder Säure eine klassische Racematspaltung mit den Verbindungen der Formel I oder ihren Zwischenprodukten durchführt.

Gegenstand der Erfindung sind auch zu Verbindungen der Formel I mesomere oder tautomere Verbindungen.

Ein weiterer Gegenstand der Erfindung sind die physiologisch verträglichen Salze der Verbindungen I, die sich durch Umsatz von Verbindungen I mit einer geeigneten Säure oder Base erhalten lassen. Geeignete Säuren und Basen sind zum Beispiel in Fortschritte der Arzneimittelforschung, 1966, Birkhäuser Verlag, Bd.10, S. 224-285, aufgelistet. Dazu zählen zum Beispiel Salzsäure, Citronensäure, Weinsäure, Milchsäure, Phosphorsäure, Methansulfonsäure, Essigsäure, Ameisensäure, Maleinsäure, Fumarsäure usw. bzw. Natriumhydroxid, Lithiumhydroxid, Kaliumhydroxid und Tris.

Unter Prodrugs werden solche Verbindungen verstanden, die in vivo in Verbindungen der allgemeinen Formel I metabolisiert werden. Typische Prodrugs sind Phosphate, Carbamate von Aminosäuren, Ester und andere.

Die Herstellung der erfindungsgemäßen Substanzen I kann auf verschiedenen Wegen erfolgen, die analog denen sind, die in WO 98/06703 für Benzimidazol und Indol gezeigt wurden, und den Syntheseschemata 1-3 skizziert wurden.

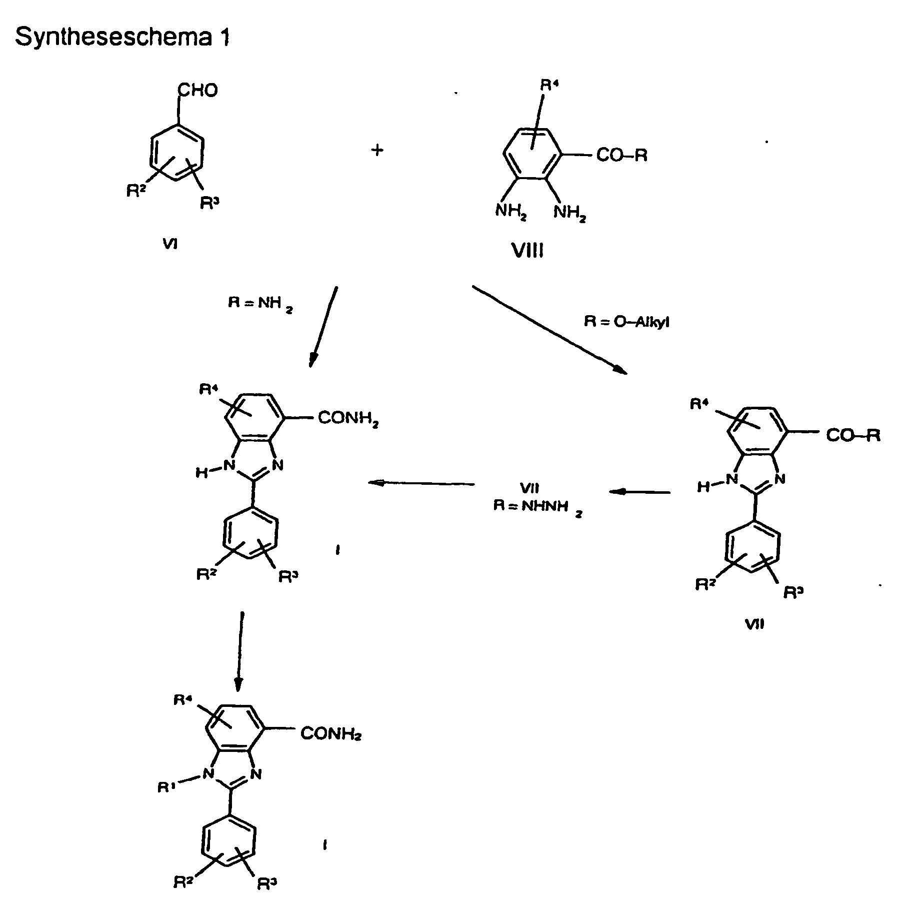

Durch Kondensation des Benzaldehyds mit Phenylendiaminen erhält man das Benzimidazol VII, wobei man bevorzugt in polaren Lösungsmitteln wie Ethanol oder Dimethylformamid und Zusatz von Säuren wie Essigsäure bei erhöhter Temperatur arbeitet, in der Regel 80-120 °C. Günstig für die Reaktion ist der Zusatz von schwachen Oxidationsmittel wie Kupfer-II-Salzen, die als wäßrige Lösung zugesetzt werden.

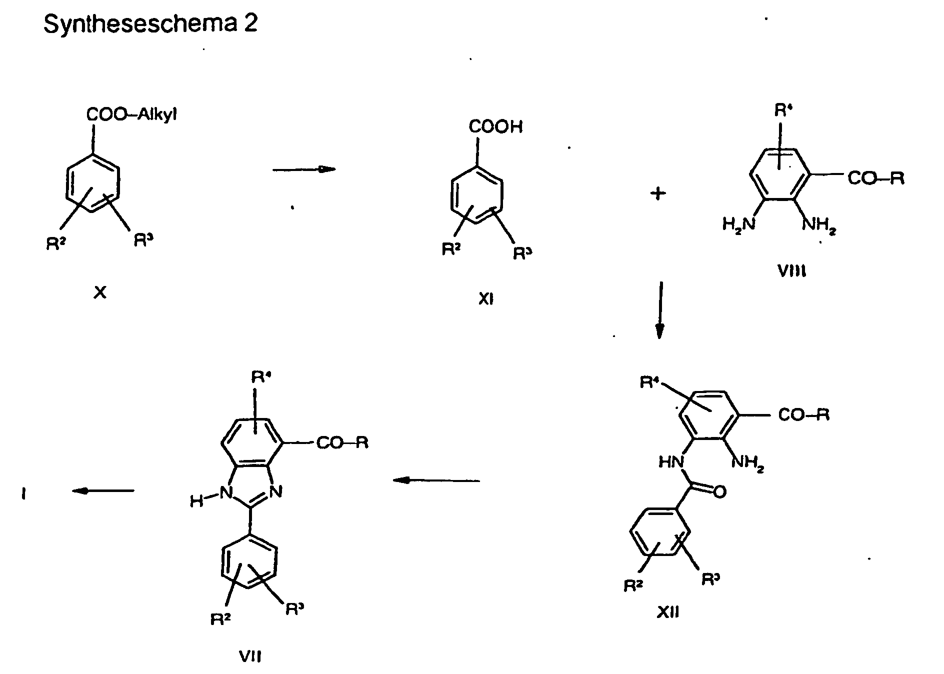

Wenn in dem Phenylendiamin VIII R = NH₂ ist, entstehen bei der Kondensation direkt erfindungsgemäße Verbindungen I. Ansonsten kann man, falls R = O-Alkyl ist, diesen Ester mit Ammoniak, bei gegebenenfalls erhöhter Temperatur und erhöhtem Druck, zum Amid I umsetzen. Alternativ kann man den Ester VIII mit Hydrazin in polaren Lösungsmitteln wie die Alkohole Butanol und Ethanol oder auch Dimethylformamid, bei erhöhten Temperaturen, vorzugsweise 80-130 °C, umsetzen, wobei ein Hydrazid VII (R = NHNH₂) anfällt, das danach noch unter reduktiven Bedingungen, wie mit Raney-Nickel in Alkoholen unter Rückfluß, zum Amid I reduziert werden kann.

Eine Einführung des Restes R1 am Benzimidazol-Rest in I (R1 = H) gelingt unter Alkylierungsbedingungen wie oben, wobei allerdings der Reaktionspartner R1-L (L = Abgangsgruppe) eingesetzt werden muß (siehe Schema 1).

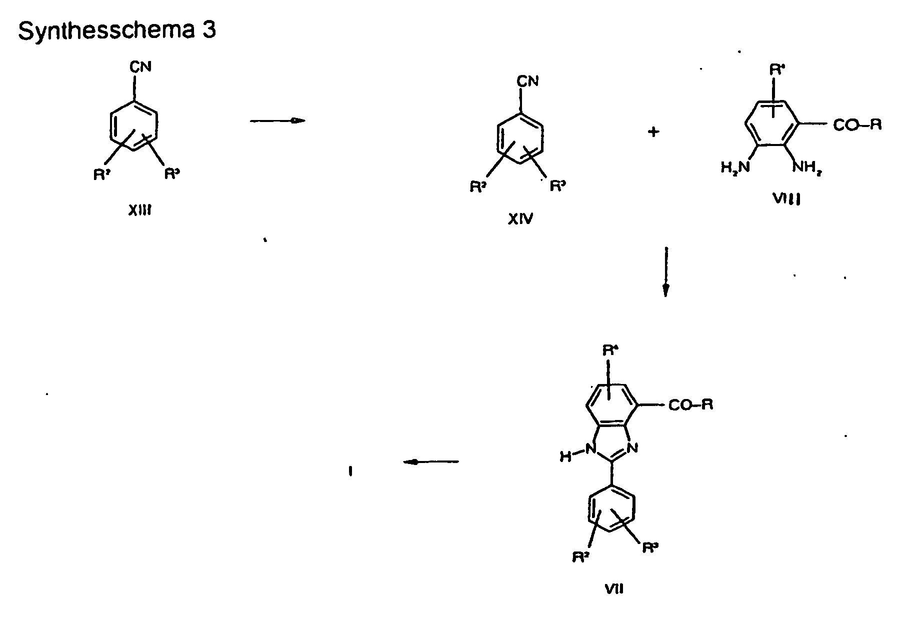

Alternativ zu den im Schema 1 gezeigten Benzaldehyden VI kann man auch Benzoesäuren wie XI (siehe Schema 2) oder Benzonitrile wie XIV (siehe Schema 3) anstelle des Benzaldehyds einsetzen. Die Herstellung dieser Derivate erfolgt analog zur Herstellung der substituierten Benzaldehyde VIII. Ausgehend von XI erfolgt die Kondensation zu VII in zwei Stufen . Zuerst wird die Benzoesäure XI mit dem Anilin VIII in einer peptidartigen Kupplung zum Amid XII umgesetzt. Dabei arbeitet man nach üblichen Bedingungen, die zum Beispiel im Houben-Weyl, Methoden der Organischen Chemie, 4.Aufl., E5, Kap. V bzw. C. R. Larock, Comprehensive Organic Transformations, VCH Publisher, 1989, Seite 972f. aufgelistet sind. Der Ringschluß zum Benzimidazol erfolgt danach bei erhöhter Temperatur, zum Beispiel 60-180 °C, mit oder ohne Lösungsmitteln wie Dimethylformamid, unter Zusatz von Säuren wie Essigsäure oder direkt in Essigsäure selbst.

Die Reaktion des Phenylendiamins VIII mit einem Benzonitril XIV erfolgt ebenfalls unter üblichen Bedingungen. Dabei kann man in Lösungsmitteln; wie Dimethylformamid unter Zusatz von Säuren oder auch in Polyphosphorsäure bei erhöhter Temperatur wie 60-200 °C arbeiten. Allerdings kann man auch die üblichen Methoden zur Herstellung von Amidinen aus Benzonitrilen anwenden, wie sie in Houben-Weyl, Methoden der organischen Chemie, E5, S. 1304f. , J. Amer. Chem. Soc. **1957**, 427 und J. Org. Chem. 1987, 1017 beschrieben sind.

Die in der vorliegenden Erfindung enthaltenen substituierten. 2-Phenylbenzimidazole und 2-Phenylindole I stellen Inhibitoren des Enzyms Poly(ADP-ribose)polymerase oder PARP (EC 2.4.2.30) dar.

Die inhibitorische Wirkung der substituierten 2-Phenylbenzimidazole und 2-Phenylindole I wurde mit einem in der Literatur bereits bekannten Enzymtest ermittelt, wobei als Wirkmaßstab ein Ki-Wert ermittelt wurde. Die 2-Phenylbenzimidazole und 2-Phenylindole I wurden in dieser Weise auf Hemmwirkung des Enzyms Poly(ADP-ribose)polymerase oder PARP (EC 2.4.2.30) gemessen.

Die substituierten 2-Phenylbenzimidazole und 2-Phenylindole der allgemeinen Formeln I stellen Inhibitoren der Poly(ADP-ribose)polymerase (PARP) bzw. wie es auch genannt wird Poly(ADP-ribose)synthase (PARS) dar und können somit zur Behandlung und Prophylaxe von Krankheiten, die mit einer erhöhten Enzymaktivität dieser Enzyme verbunden sind, dienen.

Die Verbindungen der Formeln I können zur Herstellung von Arzneimitteln zur Behandlung von Schädigungen nach Ischämien und zur Prophylaxe bei erwarteten Ischämien verschiedener Organe eingesetzt werden.

Die vorliegenden 2-Phenylbenzimidazole und 2-Phenylindole der allgemeinen Formel I können danach zur Behandlung und Prophylaxe von neurodegenerativen Krankheiten, die nach Ischämie, Trauma (Schädel-Hirntrauma), Massenblutungen, Subarachnoidal-Blutungen und Stroke auftreten, und von neurodegenerativen Krankheiten wie multipler Infarkt-Dementia, Alzheimer Krankheit, Huntington Krankheit und von Epilepsien, insbesondere von generalisierten epileptischen Anfällen, wie zum Beispiel Petit mal und tonischclonische Anfälle und partiell epileptischen Anfällen, wie Temporal Lope, und komplex-partiellen Anfällen, und weiterhin zur Behandlung und Prophylaxe von Schädigungen des Herzens nach cardialen Ischämien und Schädigungen der Nieren nach renalen Ischämien, zum Beispiel der akuten Niereninsuffizienz, des akuten Nierenversagens oder von Schädigungen, die während und nach einer Nierentransplantation auftreten, dienen. Weiterhin können die Verbindungen der allgemeinen Formel I zur Behandlung des akuten Myocardinfarkts und Schädigungen, die während und nach dessen medikamentöser Lyse auftreten (zum Beispiel mit TPA, Reteplase, Streptokinase oder mechanisch mit einem Laser oder Rotablator) und von Mikroinfarkten während und nach Herzklappenersatz, Aneurysmenresektionen und Herztransplantationen dienen. Ebenfalls können die vorliegenden 2-Phenylbenzimidazole und 2-Phenylindole I zur Behandlung einer Revascularisation kritisch verengter Koronaraterien, zum Beispiel bei der PTCA und Bypass-Operationen, und kritisch verengter peripherer Arterien, zum Beispiel Beinarterien, dienen. Zudem können die 2-Phenylbenzimidazole und 2-Phenylindole I bei der Chemotherapie von Tumoren und deren Metastasierung nützlich sein und zur Behandlung von Entzündungen und rheumatischen Erkrankungen, wie z.B. rheumatischer Arthritis dienen.

Weiterhin können die Verbindungen der allgemeinen Formel I zur Behandlung von Sepsis des Multiorganversagens wie zum Beispiel während des septischen Schocks und des "acute respiratory distress-syndroms" sowie zur Behandlung von Diabetes mellitus eingesetzt werden.

Die erfindungsgemäßen Arzneimittelzubereitungen enthalten neben den üblichen Arzneimittelhilfstoffen eine therapeutisch wirksame Menge der Verbindungen I.

Für die lokale äußere Anwendung, zum Beispiel in Puder, Salben oder Sprays, können die Wirkstoffe in den üblichen Konzentrationen enthalten sein. In der Regel sind die Wirkstoffe in einer Menge von 0,001 bis 1 Gew.-%. vorzugsweise 0,001 bis 0,1 Gew.-% enthalten.

Bei der inneren Anwendung werden die Präperationen in Einzeldosen verabreicht. In einer Einzeldosis werden pro kg Körpergewicht 0,1 bis 100 mg gegeben. Die Zubereitung können täglich in einer oder mehreren Dosierungen je nach Art und Schwere der Erkrankungen verabreicht werden.

Entsprechend der gewünschten Applikationsart enthalten die erfindungsgemäßen Arzneimittelzubereitungen neben dem Wirkstoff die üblichen Trägerstoffe und Verdünnungsmittel. Für die lokale äußere Anwendung können pharmazeutischtechnische Hilfsstoffe, wie Ethanol, Isopropanol, oxethyliertes Ricinusöl, oxethyliertes hydriertes Ricinusöl, Polyacrylsäure, Polyethylenglykol, Polyethylenglykostearat, ethoxylierte Fettalkohole, Paraffinöl, Vaseline und Wollfett, verwendet werden. Für die innere Anwendung eignen sich zum Beispiel Milchzucker, Propylenglykol, Ethanol, Stärke, Talk und Polyvinylpyrrolidon.

Ferner können Antioxidationsmittel wie Tocopherol und butyliertes Hydroxyanisol sowie butyliertes Hydroxytoluol, geschmacksverbessernde Zusatzstoffe, Stabilisierungs-, Emulgier- und Gleitmittel enthalten sein.

Die neben dem Wirkstoff in der Zubereitung enthaltenen Stoffe sowie die bei der Herstellung der pharmazeutischen Zubereitungen verwendeten Stoffe sind toxikologisch unbedenklich und mit dem jeweiligen Wirkstoff verträglich. Die Herstellung der Arzneimittelzubereitungen erfolgt in üblicher Weise, zum Beispiel durch Vermischung des Wirkstoffes mit anderen üblichen Trägerstoffen und Verdünnungsmitteln.

Die Arzneimittelzubereitungen können in verschiedenen Applikationsweisen verabreicht werden, zum Beispiel peroral, parenteral wie intravenös durch Infusion, subkutan, intraperitoneal und topisch. So sind Zubereitungsformen wie Tabletten, Emulsionen, Infusions- und Injektionslösungen, Pasten, Salben, Gele, Cremes, Lotionen, Puder und Sprays möglich.

### Beispiel 1

### 2(4-(N,N-2-(N,N-Diethylamino)eth-1-yl-methylamino)phenyl)benzimidazol-4-carbonsäureamid

a) 2(4-(N,N-2-(N,N-Diethylamino)eth-1-yl-methylamino)phenyl)-benzimidazol-4-carbonsäureethylester
   2,0 g (12 mMol) 2,3-Diaminobenzoesäureethylester wurden in 100 ml Methanol gelöst und mit 1,7 ml (27,7 mMol) Essigsäure versetzt. Anschließend wurden 2,4 g (10,1 mMol) 4(2(N,N-Diethylamino)eth-1-yl-methylamino)benzaldehyd, gelöst in 100 ml Methanol, innerhalb von 30 Minuten zugetropft. Man tropfte eine Lösung von 1,7 g (8,5 mMol) Kupfer-II-acetat in 30 ml Wasser zu und erwärmt danach alles für 50 Minuten unter Rückfluß. Man ließ die Reaktionslösung auf 50°C abkühlen und gab vorsichtig 20 ml 32%ige Salzsäure zu. Dann wurde noch eine Lösung aus 3,9 g (16,2 mMol) Natriumsulfid-Hydrat in 20 ml Wasser zugetropft und alles für 10 Minuten gerührt. Der Niederschlag wurde abgesaugt und das Filtrat durch Zugabe von wäßriger Natriumhydrogenkarbonat-Lösung alkalisiert. Diese wäßrige Phase wurde mit Essigester extrahiert, die organische Phase abgetrennt, getrocknet und im Vakuum eingeengt. Man erhielt 2,6 g des Produktes.
b) 2(4-(N,N-2-(N,N-Diethylamino)eth-1-yl-methylamino)phenyl)-benzimidazol-4-carbonsäurehydrazid
   2,6 g (6,8 mMol) des Zwischenproduktes 1 und 3,4 g (68,3 mMol) Hydrazinhydrat wurden in 70 ml n-Butanol gegeben und für 12 Stunden auf 120°C erwärmt. Danach wurde das Butanol im Vakuum entfernt. Der erhaltene Rückstand wurde zwischen Wasser und Essigester verteilt. Die organische Phase wurde abgetrennt, getrocknet und im Vakuum eingeengt. Man erhielt 1,1 g des Produktes.
c) 2(4-(N,N-2-(N,N-Diethylamino)eth-1-yl-methylamino)phenyl)-benzimidazol-4-carbonsäureamid
   Zu 1,1 g (2,9 mMol) des Zwischenproduktes 1b in 30 ml Dimethylformamid wurden 1 g Raney-Nickel gegeben und alles für 8 Stunden auf 120°C erwärmt. Das Reaktionsgemisch wurde filtriert und das Filtrat im Vakuum eingeengt. Der erhaltene Rückstand wurde zwischen Wasser und Essigester verteilt. Die organische Phase wurde abgetrennt, getrocknet und im Vakuum eingeengt. Man erhielt 0,9 g des Produktes.
   ¹H-NMR (D₆-DMSO): δ = 2,2 (6H), 2,4 (2H), 3,0 (3H). 3,5 (2H), 6,8 (2H), 7,2 (1 H), 7,6-7,8 (3H), 8,1 (2H), 9,5 (1H) und 13,2 (1H) ppm.

### Beispiel 2

### 2(4-(N,N-2-(N.N-Dimethylamino)eth-1-yl-methylamino)phenyl)benzimidazol-4-carbonsäureamid

Die Verbindung wurde analog zu den Vorschriften im Beispiel 1 hergestellt.
¹H-NMR (D₆-DMSO): δ = 2,2 (6H), 2,55 (2H), 3,1 (2H), 7,4 (1H), 7,8 (2H), 7,9 (1H), 8,1 (1H), 8,3 (1H), 8,4 (1H), 9,2 (1H) ppm.

### Beispiel 3

### 2(3(2(N,N-Dimethylamino)eth-1-yl)-4-nitrophenyl)benzimidazol-4-carbonsäureamid

a) 3(E-2-N,N-Dimethylamino-ethen-1-yl)-4-nitro-benzoesäuremethylester
   10 g (47,8 mMol) 3-Methyl-4-nitrobenzoesäureethylester und 30 ml N,N-Dimethylformamiddimethylacetal wurden in 100 ml Dimethylformamid für 8 Stunden unter Rückfluß gekocht. Anschließend wurde der Ansatz im Vakuum eingeengt. Der Rückstand wurde in 100 ml Toluol gelöst und durch Zugabe von Petrolether wurde das Produkt ausgefällt. Man erhielt 7,5 g des Produktes.
b) 3(2-N,N-Dimethylamino-eth-1-yl)-4-nitrobenzylalkohol
   Zu 7 g (26,5 mMol) des Zwischenproduktes 3a in 70 ml Ethanol wurden 2,0 g (53 mMol) Natriumborhydrid portionsweise zugegeben. Anschließend wurde alles für 30 Minuten unter Rückfluß erwärmt. Die Reaktionslösung wurde dann im Vakuum eingeengt. Der erhaltene Rückstand wurde zwischen Wasser und Essigester verteilt. Die organische Phase wurde abgetrennt, mit Wasser und mit wäßriger Vakuum eingeengt. Das so efialtene Öl wurde in Ethanol gelöst und mit etherische Chlorwasserstoff-Lösung versetzt. Das Produkt kristallisierte als Hydrochlorid aus. Man erhielt 2,5 g.
b) 3(2-N,N-Dimethylamino-eth-1-yl) 4-nitrobenzaldehyd
   2,35 g (9 mMol) des Zwischenproduktes 3b und 6,3 ml (45 mMol) Triethylamin wurden in 50 ml Dimethylsulfoxid gelost. Danach gab man 2,9 g (18 mMol) des Pyridin-Schwefeltrioxid-Adduktes portionsweise zu und rührte alles für 60 Minuten. Danach wurde der Ansatz im Vakuum eingeengt und der Rückstand zwischen Wasser und Essigester verteilt. Die organische Phase wurde noch zweimal mit Wasser gewaschen, getrocknet und im Vakuum eingeengt. Man erhielt 1,8 g des Produktes.
d) 2(3(2(N,N-Dimethylamino)eth-1-yl)-4-nitrophenyl)benzimidazol-4-carbonsäureamid

Das Zwischenprodukt 3c) wurde analog der Vorschriften aus Beispiel 1 a), b) und c) zum Produkt umgesetzt.
¹H-NMR (D₆-DMSO): δ = 1,25 (6H), 3,1 (3H), 3,2 (4H), 3,9 (2H), 7,0 (2H), 7,2 (1H), 7,6-7,9 (3H), 8,1 (2H), 9,5 (1H), 10,9 (1H) und 13,5 (breit) ppm.

Analog der Methoden, die in WO 98/06703 beschrieben wurden oder der Methoden in der vorliegenden Anmeldung beschrieben sind, können folgende Beispiele hergestellt werden:
1. 2(4-(Diethylamino)methyl)phenyl-benzimidazol-4-carbonsäureamid
2. 2(4-(Dimethylamino)methyl)phenyl-benzimidazol-4-carbonsäureamid
3. 2(4-(Pyrrolidin-1-yl)methyl)phenyl-benzimidazol-4-carbonsäureamid
4. 2(4-(Piperidin-1-yl)methyl)phenyl-benzimidazol-4-carbonsäureamid
5. 2(4-Aminomethyl)phenyl-benzimidazol-4-carbonsäureamid
6. 2(4-(Methylamino)methyl)phenyl-benzimidazol-4-carbonsäureamid
7. 2(4-(Propylamino)methyl)phenyl-benzimidazol-4-carbonsäureamid
8. 2(4(2(Diethylamino)eth-1-yl)phenyl)-benzimidazol-4-carbonsäureamid
9. 2(4(2(Dimethylamino)eth-1-yl)phenyl)-benzimidazol-4-carbonsäureamid
10. 2(4(2-Aminoeth-1-yl)phenyl)-benzimidazol-4-carbonsäureamid
11. 2(4(2(Methylamino)eth-1-yl)phenyl)-benzimidazol-4-carbonsäureamid
12. 2(4(2(Ethylamino)eth-1-yl)phenyl)-benzimidazol-4-carbonsäureamid
13. 2(4(2(Pyrrolidin-1yl)eth-1-yl)phenyl)-benzimidazol-4-carbonsäureamid
14. 2(4(2(Piperidin-1-yl)eth-1-yl)phenyl)-benzimidazol-4-carbonsäureamid
15. 2(3-(Diethylamino)methyl)phenyl-benzimidazol-4-carbonsäureamid
16. 2(3-(Dimethylamino)methyl)phenyl-benzimidazol-4-carbonsäureamid
17. 2(3-(Pyrrolidin-1-yl)methyl)phenyl-benzimidazol-4-carbonsäureamid
18. 2(3-(Piperidin-1-yl)methyl)phenyl-benzimidazol-4-carbonsäureamid
19. 2(3-Aminomethyl)phenyl-benzimidazol-4-carbonsäureamid
20. 2(3-(Methylamino)methyl)phenyl-benzimidazol-4-carbonsäureamid
21. 2(3-(n-Propylamino)methyl)phenyl-benzimidazol-4-carbonsäureamid
22. 2(3(2(Diethylamino)eth-1-yl)phenyl)-benzimidazol-4-carbonsäureamid
23. 2(3(2(Dimethylamino)eth-1-yl)phenyl)-benzimidazol-4-carbonsäureamid
24. 2(3(2-Aminoeth-1-yl)phenyl)-benzimidazol-4-carbonsäureamid
25. 2(3(2(N-Methylamino)eth-1-yl)phenyl)-benzimidazol-4-carbonsäureamid
26. 2(3(2(N-Ethylamino)eth-1-yl)phenyl)-benzimidazol-4-carbonsäureamid
27. 2(3(2(Pyrrolidin-1yl)eth-1-yl)phenyl)-benzimidazol-4-carbonsäureamid
28. 2(3(2(Piperidin-1-yl)eth-1-yl)phenyl)-benzimidazol-4-carbonsäureamid
29. 2(4-N,N-(2-Aminoeth-1-yl)methylamino)phenyl-benzimidazol-4-carbonsäureamid
30. 2(4-N-(2(Diethylamino)eth-1-yl)amino)phenyl-benzimidazol-4-carbonsäureamid
31. 2(4-N-(2(Dimethylamino)eth-1-yl)amino)phenyl-benzimidazol-4-carbonsäureamid
32. 2(4-N-(2-Aminoeth-1-yl)amino)phenyl-benzimidazol-4-carbonsäureamid
33. 2(3-N,N-(2(Dimethylamino)eth-1-yl)methylamino)phenyl-benzimidazol-4-carbonsäureamid
34. 2(3-N,N-(2-Aminoeth-1-yl)methylamino)phenyl-benzimidazol-4-carbonsäureamid
35. 2(3-N-(2(Diethylamino)eth-1-yl)amino)phenyl-benzimidazol-4-carbonsäureamid
36. 2(3-N-(2(Dimethylamino)eth-1-yl)amino)phenyl-benzimidazol-4-carbonsäureamid
37. 2(3-N-(2-Aminoeth-1-yl)amino)phenyl-benzimidazol-4-carbonsäureamid
38. 2(3-N,N-(3(Diethylamino)prop-1-yl)methylamino)phenyl-benzimidazol-4-carbonsäureamid
39. 2(3-N,N-(3(Dimethylamino)prop-1-yl)methylamino)phenyl-benzimidazol-4-carbonsäureamid
40. 2(3-N,N-(3-Aminoprop-1-yl)methylamino)phenyl-benzimidazol-4-carbonsäureamid
41. 2(3-N-(3(Diethylamino)prop-1-yl)amino)phenyl-benzimidazol-4-carbonsäureamid
42. 2(3-N-(3(Dimethylamino)prop-1-yl)amino)phenyl-benzimidazol-4-carbonsäureamid
43. 2(3-N-(3-Aminoprop-1-yl)amino)phenyl-benzimidazol-4-carbonsäureamid
44. 2 (3-N,N-(2-Pyrrolidion-1-yl-eth-1-yl)methylamino)phenyl-benzimidazol-4-carbonsäureamid
45. 2(3-N-(2(Pyrrolidion-1-yl)eth-1-yl)amino)phenyl-benzimidazol-4-carbonsäureamid
46. 2(3-N,N-(3(Pyrrolidin-1-yl)prop-1-yl)methylamino)phenyl-benzimidazol-4-carbonsäureamid
47. 2(3-N,N-(3(Piperidin-1-yl)prop-1-yl)methylamino)phenyl-benzimidazol-4-carbonsäureamid
48. 2(3-N,N-(2(Piperidin-1-yl)eth-1-yl)methylamino)phenyl-benzimidazol-4-carbonsäureamid

### Beispiel A: Hemmung des Enzyms Poly(ADP-ribose)polymerase oder PARP (EC 2.4.2.30)

Eine 96well Mikrotiterplatte (Falcon) wird mit Histonen (Type II-AS; SIGMA H7755) beschichtet. Histone werden dazu in Carbonat-Puffer (0,05 M NaHCO₃; pH 9,4) zu einer Konzentration von 50 µg/ml gelöst. Die einzelnen Wells der Mikrotiterplatte werden über Nacht mit je 100 µl dieser Histon Lösung inkubiert. Anschließend wird die Histon Lösung entfernt und die einzelnen Wells mit 200 µl einer 1%igen BSA (Bovine Serum Albumine) Lösung in Carbonat-Puffer für 2 Stunden bei Raumtemperatur inkubiert. Anschließend wird dreimal mit Waschpuffer (0,05 % Tween10 in PBS) gewaschen. Für die Enzymreaktion werden je Well 50 µl der Enzymreaktionslösung (5µl Reaktions-Puffer (1M Tris-HCl pH 8,0, 100 mM MgCl₂, 10 mM DTT,) 0,5 µl PARP (c = 0,22 µg/µl), 4 µl aktivierte DNA (SIGMA D-4522, 1mg/ml in Wasser), 40,5 µl H₂O) mit 10 µl einer Inhibitorlösung für 10 Minuten vorinkubiert. Die Enzymreaktion wird durch Zugabe von 40 µl einer Substratlösung (4 µl Reaktion-Puffer (s.o.), 8 µl NAD-Lösung (100 µM in H₂O), 28 µl H₂O) gestartet. Reaktionszeit ist 20 Minuten bei Raumtemperatur. Die Reaktion wird durch dreimaliges Waschen mit Waschpuffer (s.o.) gestoppt. Anschließend folgt eine einstündige Inkubation bei Raumtemperatur mit einem spezifischen Anti-Poly-ADP-Ribose Antikörper. Als Antikörper wurden ein monoklonaler anti-Poly-(ADP-ribose) Antikörper "10H" (Kawamaitsu H. et al. (1984) Monoclonal antibodies to poly (adenosine diphosphate ribose) recognize different structures. Biochemistry 23, 3771-3777) verwendet. Polyklonale Antikörper können ebenso verwendet werden.

Die Antikörper wurden in einer 1:5000 Verdünnung in Antikörper-Puffer (1%BSA in PBS; 0,05 % Tween20) eingesetzt. Nach dreimaligem Waschen mit Waschpuffer folgt eine einstündige Inkubation bei Raumtemperatur mit dem sekundärem Antikörper. Hier wurden für den monoklonalen Antikörper ein anti-Maus-IgG gekoppelt mit Peroxidase (Boehringer Mannheim) und für den Kaninchen Antikörper ein anti-Rabbit-IgG gekoppelt mit Peroxidase (SIGMA A-6154) jeweils in einer 1:10000 Verdünnung in Antikörperpuffer verwendet. Nach dreimaligem Waschen mit Waschpuffer erfolgt die Farbreaktion unter Verwendung von 100 µl/Well Farbreagenz (SIGMA, TMB-Fertigmix, T8540) für ca. 15 min. bei Raumtemperatur. Die Farbreaktion wird durch Zugabe von 100 µl 2M H₂SO₄ gestoppt. Danach wird sofort gemessen (450 nm gegen 620 nm; ELISA Platten Lesegerät "Easy Reader" EAR340AT, SLT-Labinstruments, Österreich). Der IC₅₀-Wert eines zu messenden Inhibitors liegt bei der Inhibitorkonzentration, wo eine halbmaximale Farbkonzentrationsänderung auftritt. Der Kᵢ-Wert entspricht der Inhibitonskonstante. Folgende Kᵢ-Werte wurden bestimmt:
Beispiel 1: 16 nM
Beispiel 2: 10 nM
Beispiel 3: 4 nM

### Beispiel B: Bestimmung der Wasserlöslichkeit

Eine zu messende Verbindung wird direkt in einem festgelegten Volumen Wasser gelöst und die entstandene Lösung mit einer Natriumacetat-Lösung auf pH 5 bis 6 eingestellt, so daß die zu prüfende Konzentration des Wirkstoffs erreicht wird. Falls die Meßsubstanz nicht als wasserlösliches Salz vorliegt, wurde diese in möglichst wenig Dimethylsulfoxid gelöst und anschließend mit Wasser verdünnt (Endkonzentration an Dimethylsulfoxid ≤ 1 %), wonach auch hier der pH-Wert noch eingestellt wurde. Der potente PARP-Inhibitor NU 1076 (WO 97/04771) zeigte hier eine Löslichkeit < 0,01 %, wogegen die erfindungsgemäßen Beispiele eine Löslichkeit > 0,5 % aufweisen.

### Beispiel C: Test von PARP-Inhibitoren in einem zellulären Assay

Zum Test der Wirkung von PARP-Inhibitoren werden eukaryontische Zellinien mit Chemikalien so behandelt, daß die DNA der Zellinie geschädigt und dadurch das in den Zellen vorhandene PARP-Enzym aktiviert wird. Durch die Aktivierung des Enzym werden Ketten von poly-ADP-Ribose (PAR) auf Proteinen gebildet. Diese Ketten werden von einem spezifischen Antikörper gebunden. Dieser wird wiederum von einem zweiten Antikörper gebunden, der mit einer fluoreszenten Markierung versehen ist. Die Fluoreszenz wird mit einem Fluoreszenzscanner gemessen und verhält sich zur Aktivität des Enzyms PARP proportional. PARP-Inhibitoren lassen sich an einer Abschwächung des Fluoreszenzsignals erkennen. Um Verfälschungen der Ergebnisse durch unterschiedliche Zellzahlen zu verhindern, wird die DNA der Zellen mit einem weiteren Farbstoff markiert und dessen Fluoreszenz ebenfalls im Fluoreszenzscanner bestimmt.

400000 Zellen der humanen Zellinie C4I werden in Zellkulturplatten mit 24 Kavitäten in RPMI-Medium mit 10% fötalen Rinderserum bei 37°C, 5 % CO₂ bis zum Erreichen eines dichten Zellrasens bebrütet. Die Zellen werden mit DMEM gewaschen und die zu testenden PARP-Inhibitoren in verschiedenen Konzentrationen in DMEM zugegeben. Nach einer Inkubation für 20 min bei 37°C wird mit Wasserstoffperoxid eine Konzentration von 1 mM eingestellt und weitere 10 min bei 37°C inkubiert. Zur Kontrolle werden Zellen in einigen Kavitäten nicht mit Wasserstoffperoxid behandelt (keine PARP-Aktivierung) oder erhalten keinen Inhibitor (maximale PARP-Aktivierung). Die Zellen werden einmal mit PBS gewaschen und durch Zugabe von auf -20°C vorgekühltem Methanol/Aceton Gemisch (7 Teile Methanol, 3 Teile Aceton) 10 min bei -20°C fixiert. Danach werden die Zellen getrocknet, durch Zugabe von PBS für 10min bei Zimmertemperatur rehydratisiert und unspezifische Bindungsstellen in PBS mit 0,05 % Tween20 und 5 % Trockenmilchpulver für 30 min bei Zimmertemperatur blockiert. Der Maus anti-PAR Antikörper wird in einer Konzentration von 20 µg/ml in PBS mit 0,05 % Tween20 und 5 % Trockenmilchpulver zugegeben und 1 h bei 37°C inkubiert. Nicht gebundener Antikörper wird durch fünfmaliges Waschen mit PBS für jeweils 5 min entfernt. Anschließend wird mit einem verdünnten Ziege anti-Maus FITC-gekoppelten Zweitantikörper (Verdünnung 1:50 in PBS mit 0,05 % Tween20, 5 % Trockenmilchpulver und 1 µg/ml DAPI (4',6-Diamidino-2-Phenylindol)) für 30 min bei 37°C inkubiert. Nicht gebundener Antikörper wird durch fünfmaliges Waschen mit PBS für jeweils 5min entfernt. Die FITC- und DAPI-Fluoreszenzen werden an mehreren Stellen der Kavitäten mit Hilfe eines Fluoreszenzscanners gemessen. Zur Auswertung wird das FITC-Signal auf das DAPI-Signal normiert. Die Berechnung der IC50-Werte erfolgt nach halblogarithmischer Auftragung der normierten Werte der verschiedenen Inhibitorkonzentrationen. Folgende IC₅₀-Werte wurden bestimmt:
Beispiel 1: 115 nM
Beispiel 2: 119 nM
Beispiel 3: 118 nM

## Patentansprüche

1. Verwendung von Verbindungen der Formel I 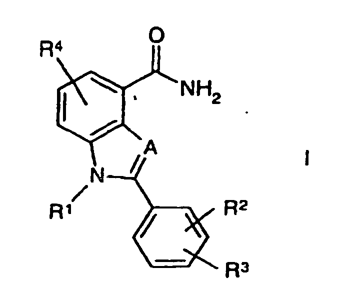 worin
A N oder CH bedeutet,
R¹ Wasserstoff, verzweigtes und unverzweigtes C₁-C₆-Alkyl, wobei ein C-Atom des Alkyl-Restes noch OR¹¹ tragen kann, wobei
R¹¹ Wasserstoff oder C₁-C₄-Alkyl bedeutet, und
R² Wasserstoff, Chlor, Fluor, Brom, Jod, verzweigtes und unverzweigtes C₁-C₆-Alkyl, Nitro, CF₃, CN, NR²¹R²², NH-CO-R²³, OR²¹, wobei
R²¹ und R²² unabhängig voneinander Wasserstoff oder C₁-C₄-Alkyl bedeuten und
R²³ Wasserstoff, C₁-C₄-Alkyl oder Phenyl bedeuten, und
R³ -NR³¹R³², -(CH₂)_{q}-NR³³R³⁴ bedeutet, wobei q 1, 2 oder 3 sein kann,
R³¹ bedeutet Wasserstoff, C₁-C₆-Alkyl, (CH₂)ᵣNR³³R³⁴
R₃₂ bedeutet (CH₂)ᵣNR³³R³⁴,
worin bei R³¹ und R³² unabhängig voneinander r 2, 3, 4, 5 oder 6 bedeutet und R³³ und R³⁴ unabhängig voneinander Wasserstoff, C₁-C₆-Alkyl, zusammen mit dem Stickstoff gleich einem Ring 3 bis 8 Atomen, der ein zusätzliches Heteroatom ausgewählt aus O, N-C₁-C₄-Alkyl, N-C₀-C₂-Phenyl oder NH tragen kann, Phenyl-C₁-C₄-Alkyl, wobei der Phenylring mit bis zu 3 gleichen oder verschiedenen Substituenten ausgewählt aus der Gruppe C₁-C₆-Alkyl, Halogen, Nitro, SO₂NR³⁵R³⁶ (mit R³⁵, R³⁶ unabhängig voneinander gleich Wasserstoff, C₁-C₄-Alkyl oder zusammen mit dem Stickstoff gleich einem Ring 3 bis 8 Atomen, der ein zusätzliches Heteroatom ausgewählt aus O, S, SO₂, N-C₁-C₄-Alkyl, N-C₀-C₂-Phenyl oder NH tragen kann), C₁-C₄-Alkoxy, S(O)₀₋₂-R³⁷ (mit R³⁷ gleich Wasserstoff, C₁-C₄-Alkyl), CF₃, (CH₂)₀₋₄-COR³⁷, (CH₂)₀₋₄NR³⁵R³⁶, (CH₂)₀₋₄CONR³⁵R³⁶, (CH₂)₀₋₄OR³⁷-CH₂COOR³⁷ substituiert sein kann,
R⁴ Wasserstoff, verzweigtes und unverzweigtes C₁-C₆-Alkyl, Chlor, Brom, Fluor, Nitro, Cyano, NR⁴¹R⁴², NH-CO-R⁴³, OR⁴¹, wobei
R⁴¹ und R⁴² unabhängig voneinander Wasserstoff oder C₁-C₄-Alkyl bedeuten und
R⁴³ C₁-C₄-Alkyl oder Phenyl bedeuten, und
sowie ihre tautomeren Formen, möglichen enantiomeren und diastereomeren Formen, deren Phosphate, Carbamate von Aminosäuren, Ester, sowie mögliche physiologisch verträgliche Salze
zur Herstellung von Arzneimitteln zur Behandlung von Krankheiten, bei denen pathologisch erhöhte Aktivitäten von PARP auftreten.

2. Verwendung von Verbindungen nach Anspruch 1, wobei R² in 3-Position und R³ in 4-Position oder R² in 4-Position und R³ in 3-Position zum Benzimidazolring steht.

3. Verwendung von Verbindungen nach einem der Ansprüche 1 oder 2, wobei R¹ und R⁴ Wasserstoff bedeuten.

4. Verwendung von Verbindungen nach einem der Ansprüche 1 bis 3, wobei
R² Wasserstoff, verzweigtes oder unverzweigtes C₁-C₆-Alkyl, Nitro, CN, NH₂, O-C₁-C₄-Alkyl bedeutet.

5. Verwendung von Verbindungen nach einem der Ansprüche 1 bis 4, wobei
R³ (CH₂)_{1,2}NR³⁵R³⁶ und N(R³⁷)-(CH₂)₂₋₃NR³⁵R³⁶ bedeutet, worin R³⁷ Wasserstoff und C₁-C₄-Alkyl sein kann, R³⁵ und R³⁶ unabhängig voneinander Wasserstoff und C₁-C₄-Alkyl und zusammen als NR³⁵R³⁶ auch Piperidin, Pyrrolidin, Azepin und Piperazin sein können, wobei das Piperazin am zweiten N-Atom noch mit Wasserstoff oder C₁-C₄-Alkyl substituiert sein kann.

6. Verwendung von Verbindungen nach einem der Ansprüche 1 bis 5, wobei R² Wasserstoff und A Stickstoff bedeutet.

7. Verwendung von Verbindungen der Formel I nach einem der Ansprüche 1 bis 6 zur Herstellung von Arzneimitteln zur Behandlung von neurodegenerativen Krankheiten und neuronalen Schädigungen.

8. Verwendung nach Anspruch 7 zur Behandlung von solchen neurodegenerativen Krankheiten und neuronalen Schädigungen, die durch Ischämie, Trauma oder Massenblutungen ausgelöst werden.

9. Verwendung nach Anspruch 8 zur Behandlung des Schlaganfalls und des Schädel-Hirntraumas.

10. Verwendung nach Anspruch 8 zur Behandlung der Alzheimerschen Krankheit der Parkinsonsche Krankheit und der Huntington-Krankheit.

11. Verwendung von Verbindungen der Formel I nach einem der Ansprüche 1 bis 6 zur Herstellung von Arzneimitteln zur Behandlung oder Prophylaxe von Schädigungen durch Ischämien.

12. Verwendung von Verbindungen der Formel I nach einem der Ansprüche 1 bis 6 zur Herstellung von Arzneimitteln zur Behandlung von Epilepsien, insbesondere von generalisierten epileptischen Anfällen, wie zum Beispiel Petit mal und tonisch-clonische Anfälle und partiell epileptischen Anfällen, wie Temporal Lope, und komplex-partiellen Anfällen.

13. Verwendung von Verbindungen der Formel I nach einem der Ansprüche 1 bis 6 zur Herstellung von Arzneimitteln zur Behandlung von Schädigungen der Nieren nach renalen Ischämien und zur Behandlung während und nach Nierentransplantationen.

14. Verwendung von Verbindungen der Formel I nach einem der Ansprüche 1 bis 6 zur Herstellung von Arzneimitteln zur Behandlung von Schädigungen des Herzens nach cardialen Ischämien.

15. Verwendung von Verbindungen der Formel I nach einem der Ansprüche 1 bis 6 zur Herstellung von Arzneimitteln zur Behandlung von Mikroinfarkten wie zum Beispiel während und nach Herzklappenersatz, Aneurysmenresektionenen und Herztransplantationen.

16. Verwendung von Verbindungen der Formel I nach einem der Ansprüche 1 bis 6 zur Herstellung von Arzneimitteln zur Behandlung bei einer Revasculariation kritischer verengter Koronararterien wie zum Beispiel bei PTCA und Bypass-Operationen oder kritisch verengter peripherer Arterien, insbesondere Beinarterien.

17. Verwendung von Verbindungen der Formel I nach einem der Ansprüche 1 bis 6 zur Herstellung von Arzneimitteln zur Behandlung des akuten Myocardinfarktes und von Schädigungen während und nach dessen medikamentöser oder mechanischer Lyse.

18. Verwendung von Verbindungen der Formel I nach einem der Ansprüche 1 bis 6 zur Herstellung von Arzneimitteln zur Behandlung von Tumoren und deren Metastasierung.

19. Verwendung von Verbindungen der Formel I nach Anspruch 6 zur Herstellung von Arzneimitteln zur Behandlung von Sepsis des Multiorganversagens wie zum Beispiel während des septischen Schocks und des "acute respiratory distress-syndroms".

20. Verwendung von Verbindungen der Formel I nach einem der Ansprüche 1 bis 6 zur Herstellung von Arzneimitteln zur Behandlung von immunologischen Kranheiten wie Entzündungen und rheumatische Erkrankungen, wie zum Beispiel rheumatoide Arthritis.

21. Verwendung von Verbindungen der Formel I nach Anspruch 6 zur Herstellung von Arzneimitteln zur Behandlung von Diabetes mellitus.

22. Verbindungen, ausgewählt aus der Gruppe bestehend aus 2(4-(N,N-2-(N,N-Diethylamino)eth-1-yl-methylamino)phenyl)-benzimidazol-4-carbonsäureamid, 2(d-(N,N-2-(N,N-Dimethylamino)eth-1-yl-methylamino)phenyl)benzimidazol-4-carbonsäureamid, 2(3(2(N,N-Dimethylamino)eth-1-yl)-4-nitrophenyl)-benzimidazol-4-carbonsäureamid,
deren Phosphate, Carbamate von Aminosäuren, Ester oder Salze.

## Claims

1. Use of compounds of formula I 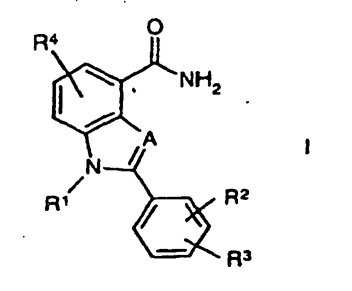 wherein
A represents N or CH,
R¹ represents hydrogen, branched and unbranched C₁-C₆-alkyl, wherein a carbon atom of the alkyl radical may further carry OR¹¹, wherein
R¹¹ represents hydrogen or C₁-C₄-alkyl, and
R² represents hydrogen, chlorine, fluorine, bromine, iodine, branched and unbranched C₁-C₆-alkyl, nitro, CF₃, CN, NR²¹R²², NH-CO-R²³, OR²¹, wherein
R²¹ and R²² independently of one another represent hydrogen or C₁-C₄-alkyl and
R²³ represents hydrogen, C₁-C₄-alkyl or phenyl, and
R³ represents -NR³¹R³², -(CH₂)_{q}-NR³³R³⁴, wherein q may be 1, 2 or 3,
R³¹ represents hydrogen, C₁-C₆-alkyl, (CH₂)ᵣNR³³R³⁴,
R³² represents (CH₂)ᵣNR³³R³⁴,
wherein in R³¹ and R³² independently of one another r represents 2, 3, 4, 5 or 6 and R³³ and R³⁴ independently of one another represent hydrogen, C₁-C₆-alkyl, together with the nitrogen a ring having from 3 to 8 atoms which may carry an additional hetero atom selected from O, N-C₁-C₄-alkyl, N-C₀-C₂-phenyl or NH; phenyl-C₁-C₄-alkyl, wherein the phenyl ring may be substituted by up to 3 identical or different substituents selected from the group C₁-C₆-alkyl, halogen, nitro, SO₂NR³⁵R³⁶ (where R³⁵ and R³⁶ independently of one another are hydrogen, C₁-C₄-alkyl or together with the nitrogen are a ring having from 3 to 8 atoms which may carry an additional hetero atom selected from O, S, SO₂, N-C₁-C₄-alkyl, N-C₀-C₂-phenyl or NH), C₁-C₄-alkoxy, S(O)₀₋₂-R³⁷ (where R³⁷ is hydrogen, C₁-C₄-alkyl), CF₃, (CH₂)₀₋₄-COR³⁷, (CH₂)₀₋₄NR³⁵R³⁶, (CH₂)₀₋₄CONR³⁵R³⁶, (CH₂)₀₋₄OR³⁷-CH₂COOR³⁷,
R⁴ represents hydrogen, branched and unbranched C₁-C₆-alkyl, chlorine, bromine, fluorine, nitro, cyano, NR⁴¹R⁴², NH-CO-R⁴³, OR⁴¹, wherein
R⁴¹ and R⁴² independently of one another represent hydrogen or C₁-C₄-alkyl and
R⁴³ represents C₁-C₄-alkyl or phenyl,
and their tautomeric forms, possible enantiomeric and diastereoisomeric forms, their phosphates, carbamates of amino acids, esters and possible physiologically acceptable salts,
in the preparation of medicaments for the treatment of diseases in which pathologically increased activities of PARP occur.

2. Use of compounds according to claim 1 wherein R² is in the 3-position and R³ is in the 4-position or R² is in the 4-position and R³ is in the 3-position with respect to the benzimidazole ring.

3. Use of compounds according to either claim 1 or claim 2 wherein R¹ and R⁴ represent hydrogen.

4. Use of compounds according to any one of claims 1 to 3 wherein
R² represents hydrogen, branched or unbranched C₁-C₆-alkyl, nitro, CN, NH₂, O-C₁-C₄-alkyl.

5. Use of compounds according to any one of claims 1 to 4 wherein
R³ represents (CH₂)_{1,2}NR³⁵R³⁶ and N (R³⁷)-(CH₂)₂₋₃NR³⁵R³⁶, wherein R³⁷ may be hydrogen and C₁-C₄-alkyl, R³⁵ and R³⁶ independently of one another may be hydrogen and C₁-C₄-alkyl and, together as NR³⁵R³⁶, may also be piperidine, pyrrolidine, azepine and piperazine, wherein the piperazine may further be substituted on the second nitrogen atom by hydrogen or C₁-C₄-alkyl.

6. Use of compounds according to any one of claims 1 to 5 wherein R² represents hydrogen and A represents nitrogen.

7. Use of compounds of formula I according to any one of claims 1 to 6 in the preparation of medicaments for the treatment of neurodegenerative diseases and neuronal damage.

8. Use according to claim 7 in the treatment of neurodegenerative diseases and neuronal damage caused by ischaemia, trauma or mass haemorrhages.

9. Use according to claim 8 in the treatment of stroke and craniocerebral trauma.

10. Use according to claim 8 in the treatment of Alzheimer's disease and Parkinson's disease and Huntington's disease.

11. Use of compounds of formula I according to any one of claims 1 to 6 in the preparation of medicaments for the treatment or prophylaxis of damage caused by ischaemia.

12. Use of compounds of formula I according to any one of claims 1 to 6 in the preparation of medicaments for the treatment of epilepsy, in particular of generalised epileptic attacks, such as, for example, petit mal and tonic-clonic attacks, and partial epileptic attacks, such as temporal lobe and complex partial attacks.

13. Use of compounds of formula I according to any one of claims 1 to 6 in the preparation of medicaments for the treatment of kidney damage following renal ischaemia and for treatment during and after kidney transplants.

14. Use of compounds of formula I according to any one of claims 1 to 6 in the preparation of medicaments for the treatment of damage to the heart after cardiac ischaemia.

15. Use of compounds of formula I according to any one of claims 1 to 6 in the preparation of medicaments for the treatment of microinfarcts, such as, for example, during and after heart valve replacement, aneurysm resections and heart transplants.

16. Use of compounds of formula I according to any one of claims 1 to 6 in the preparation of medicaments for treatment in the case of revascularisation of critically narrowed coronary arteries, such as, for example, in PTCA and bypass operations, or of critically narrowed peripheral arteries, in particular leg arteries.

17. Use of compounds of formula I according to any one of claims 1 to 6 in the preparation of medicaments for the treatment of acute myocardial infarct and of damage during and after the medicinal or mechanical lysis thereof.

18. Use of compounds of formula I according to any one of claims 1 to 6 in the preparation of medicaments for the treatment of tumours and the metastasis thereof.

19. Use of compounds of formula I according to claim 6 in the preparation of medicaments for the treatment of sepsis of multi-organ failure, such as, for example, during septic shock and acute respiratory distress syndrome.

20. Use of compounds of formula I according to any one of claims 1 to 6 in the preparation of medicaments for the treatment of immunological diseases, such as inflammations and rheumatic diseases, such as, for example, rheumatoid arthritis.

21. Use of compounds of formula I according to claim 6 in the preparation of medicaments for the treatment of diabetes mellitus.

22. Compounds selected from the group consisting of 2-(4-N,N-2-(N,N-diethylamino)eth-1-yl-methylamino)phenyl)-benzimidazole-4-carboxamide, 2-(4-(N,N-2-(N,N-dimethylamino)eth-1-yl-methylamino)phenyl)-benzimidazole-4-carboxamide, 2-(3-(2-(N,N-dimethylamino)eth-1-yl)-4-nitrophenyl)benzimidazole-4-carboxamide, their phosphates, carbamates of amino acids, esters or salts.

## Revendications

1. Utilisation de composés de formule I 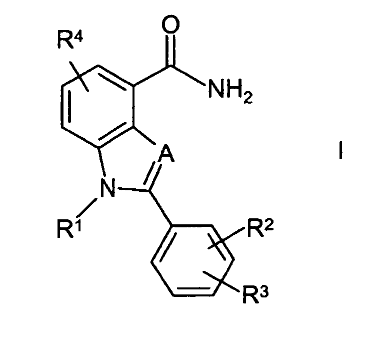 où
A représente N ou CH,
R¹ représente l'hydrogène, C₁-C₆-alkyle ramifié ou non ramifié, où un atome C du groupement alkyle peut porter encore OR¹¹, où
R¹¹ représente l'hydrogène ou C₁-C₄-alkyle, et
R² représente l'hydrogène, le chlore, le fluor, le brome, l'iode, C₁-C₆-alkyle ramifié et non ramifié, nitro, CF₃, CN, NR²¹R²², NH-CO-R²³, OR²¹, où
R²¹ et R²² représentent indépendamment l'un de l'autre l'hydrogène ou C₁-C₄-alkyle et
R²³ représente l'hydrogène, C₁-C₄-alkyle ou phényle,
et
R³ représente -NR³¹R³², -(CH₂)_{q}-NR³³R³⁴, où q peut être 1, 2 ou 3,
R³¹ représente l'hydrogène, C₁-C₆-alkyle, (CH₂)ᵣNR³³R³⁴
R³² représente (CH₂)ᵣNR³³R³⁴,
où dans R³¹ et R³², indépendamment l'un de l'autre, r représente 2, 3, 4, 5 ou 6 et R³³ et R³⁴ représentent indépendamment l'un de l'autre l'hydrogène, C₁-C₆-alkyle, représentent avec l'azote un cycle à 3 à 8 atomes, qui peut porter un hétéroatome supplémentaire choisi parmi O, N-C₁-C₄-alkyle, N-C₀-C₂-phényle ou NH, phényl-C₁-C₄-alkyle, où le cycle phényle peut être substitué avec jusqu'à 3 substituants identiques ou différents choisis dans le groupe C₁-C₆-alkyle, halogène, nitro, SO₂NR³⁵R³⁶ (avec R³⁵, R³⁶ représentant indépendamment l'un de l'autre l'hydrogène, C₁-C₄-alkyle ou avec l'azote un cycle à 3 à 8 atomes, qui peut porter un hétéroatome supplémentaire choisi parmi O, S, SO₂, N-C₁-C₄-alkyle, N-C₀-C₂-phényle ou NH), C₁-C₄-alcoxy, S(O)₀₋₂-R³⁷ (avec R³⁷ représentant l'hydrogène, C₁-C₄-alkyle) CF₃, (CH₂)₀₋₄-COR³⁷, (CH₂)₀₋₄NR³⁵R³⁶, (CH₂)₀₋₄CONR³⁵R³⁶, (CH₂)₀₋₄OR³⁷-CH₂COOR³⁷,
R⁴ représente l'hydrogène, C₁-C₆-alkyle ramifié ou non ramifié, le chlore, le brome, le fluor, nitro, cyano, NR⁴¹R⁴², NH-CO-R⁴³, OR⁴¹, où
R⁴¹ et R⁴² représentent indépendamment l'un de l'autre l'hydrogène ou C₁-C₄-alkyle et
R⁴³ représente C₁-C₄-alkyle ou phényle, et
ainsi que leurs formes tautomères, leurs formes énantiomères et diastéréoisomères possibles, leurs phosphates, carbamates d'aminoacides, leurs esters ainsi que leurs sels physiologiquement acceptables possibles pour la production de médicaments pour le traitement des maladies dans lesquelles il apparaît des activités pathologiquement augmentées de PARP.

2. Utilisation de composés selon la revendication 1, où R² est situé en position 3 et R³ en position 4 ou R² est situé en position 4 et R³ en position 3 par rapport au cycle benzimidazole.

3. Utilisation de composés selon l'une des revendications 1 ou 2, où R¹ et R⁴ représentent l'hydrogène.

4. Utilisation de composés selon l'une des revendications 1 à 3, où
R² représente l'hydrogène, C₁-C₆-alkyle ramifié ou non ramifié, nitro, CN, NH₂, O-C₁-C₄-alkyle.

5. Utilisation de composés selon l'une des revendications 1 à 4, où
R³ représente (CH₂)_{1,2}NR³⁵R³⁶ et N(R³⁷)-(CH₂)₂₋₃NR³⁵R³⁶, où R³⁷ peut être l'hydrogène et C₁-C₄-alkyle, R³⁵ et R³⁶ peuvent être indépendamment l'un de l'autre l'hydrogène et C₁-C₄-alkyle et avec NR³⁵R³⁶ également la pipéridine, la pyrrolidine, l'azépine et la pipérazine, où la pipérazine peut être substituée encore sur le deuxième atome N avec l'hydrogène ou C₁-C₄-alkyle.

6. Utilisation de composés selon l'une des revendications 1 à 5, où R² représente l'hydrogène et A représente l'azote.

7. Utilisation de composés de formule I selon l'une des revendications 1 à 6 pour la production de médicaments pour le traitement des maladies neurodégénératives et des lésions neuronales.

8. Utilisation selon la revendication 7 pour le traitement des maladies neurodégénératives et des lésions neuronales qui sont déclenchées par une ischémie, un traumatisme ou des hémorragies massives.

9. Utilisation selon la revendication 8 pour le traitement de l'apoplexie cérébrale et du traumatisme craniocérébral.

10. Utilisation selon la revendication 8 pour le traitement de la maladie d'Alzheimer, de la maladie de Parkinson et de la maladie de Huntington.

11. Utilisation de composés de formule I selon l'une des revendications 1 à 6 pour la production de médicaments pour le traitement ou la prophylaxie des lésions dues à des ischémies.

12. Utilisation de composés de formule I selon l'une des revendications 1 à 6 pour la production de médicaments pour le traitement des épilepsies, en particulier des crises épileptiques généralisées, comme par exemple le petit mal et les crises tonico-cloniques et des crises épileptiques partielles, comme Temporal Lope, et des crises partielles complexes.

13. Utilisation de composés de formule I selon l'une des revendications 1 à 6 pour la production de médicaments pour le traitement des lésions des reins après des ischémies rénales et pour le traitement pendant et après des transplantations rénales.

14. Utilisation de composés de formule I selon l'une des revendications 1 à 6 pour la production de médicaments pour le traitement des lésions du coeur après des ischémies cardiaques.

15. Utilisation de composés de formule I selon l'une des revendications 1 à 6 pour la production de médicaments pour le traitement des micro-infarctus, comme par exemple pendant et après un remplacement de valves cardiaques, des résections d'anévrisme et des transplantations cardiaques.

16. Utilisation de composés de formule I selon l'une des revendications 1 à 6 pour la production de médicaments pour le traitement lors d'une revascularisation d'artères coronaires rétrécies de manière critique comme par exemple au cours de PTCA et d'opérations de pontage ou d'artères périphériques rétrécies de manière critique, en particulier d'artères des jambes.

17. Utilisation de composés de formule I selon l'une des revendications 1 à 6 pour la production de médicaments pour le traitement de l'infarctus du myocarde aigu et des lésions pendant et après sa lyse médicamenteuse ou mécanique.

18. Utilisation de composés de formule I selon l'une des revendications 1 à 6 pour la production de médicaments pour le traitement des tumeurs et de leurs métastases.

19. Utilisation de composés de formule I selon la revendication 6 pour la production de médicaments pour le traitement de la septicémie de l'insuffisance d'organes multiples comme par exemple pendant le choc septique et le "acute respiratory distress-syndrom".

20. Utilisation de composés de formule I selon l'une des revendications 1 à 6 pour la production de médicaments pour le traitement des maladies immunologiques comme les inflammations et les maladies rhumatismales, comme par exemple la polyarthrite rhumatoïde.

21. Utilisation de composés de formule I selon la revendication 6 pour la production de médicaments pour le traitement du diabète sucré.

22. Composés choisis dans le groupe consistant en 2(4-(N,N-2-(N,N-diéthylamino)éth-1-yl-méthylamino)phényl)-benzimidazole-4-carboxamide, 2(4-(N,N-2-(N,N-diméthylamino)éth-1-yl-méthylamino)-phényl)benzimidazole-4-carboxamide, 2(3(2(N,N-diméthylamino)éth-1-yl)-4-nitrophényl)-benzimidazole-4-carboxamide, leurs phosphates, carbamates d'aminoacides, esters ou sels.
